(19) Europäisches Patentamt
European Patent Office
Office européen des brevets

(11) Veröffentlichungsnummer: **0 253 302 B1**

# (12) EUROPÄISCHE PATENTSCHRIFT

(45) Veröffentlichungstag der Patentschrift: **14.09.94**

(51) Int. Cl.[5]: **C07D 213/16**, C07D 333/08, C07D 307/36, C07D 239/26, A61K 31/44, A61K 31/38, A61K 31/34, A61K 31/505

(21) Anmeldenummer: **87109891.9**

(22) Anmeldetag: **08.07.87**

Die Akte enthält technische Angaben, die nach dem Eingang der Anmeldung eingereicht wurden und die nicht in dieser Patentschrift enthalten sind.

## (54) Tetrahydronaphthalin- und Indanderivate, deren Herstellung und Verwendung für pharmazeutische Präparate.

(30) Priorität: **15.07.86 CH 2826/86**

(43) Veröffentlichungstag der Anmeldung:
**20.01.88 Patentblatt 88/03**

(45) Bekanntmachung des Hinweises auf die Patenterteilung:
**14.09.94 Patentblatt 94/37**

(84) Benannte Vertragsstaaten:
**AT BE CH DE ES FR GB GR IT LI LU NL SE**

(56) Entgegenhaltungen:
**EP-A- 0 084 667**
**EP-A- 0 098 591**
**EP-A- 0 176 032**
**EP-A- 0 176 033**

(73) Patentinhaber: **F. HOFFMANN-LA ROCHE AG**
**Postfach 3255**
**CH-4002 Basel (CH)**

(72) Erfinder: **Klaus, Michael, Dr.**
**Am Hellenrain 6**
**D-7858 Weil/Rhein (DE)**
Erfinder: **Weiss, Ekkehard, Dr.**
**Unterer Baselblick 21B**
**D-7854 Inzlingen (DE)**

(74) Vertreter: **Lederer, Franz, Dr. et al**
**Lederer, Keller & Riederer**
**Patentanwälte**
**Prinzregentenstrasse 16**
**D-80538 München (DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Tetrahydronaphthalin- und Indanderivate der allgemeinen Formel

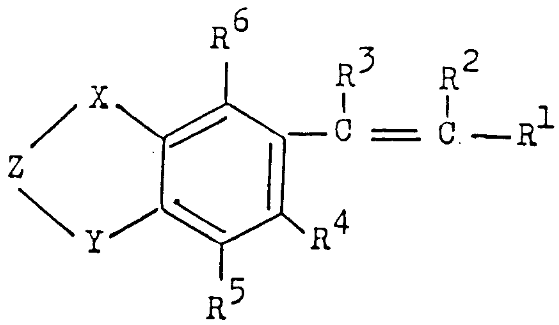

I

worin X und Y $-CH_2-$ oder $>C(CH_3)_2$; Z einen Rest $-CHR^8-$, $>CO$, $>CR^8OR^7$, $-CHR^8-CHR^8-$, $-CHOR^7-CH_2-$, $-CO-CHOR^7-$ oder $-CHOR^7-CHOR^7-$darstellt; $R^1$ Pyridyl darstellt, $R^2$ und $R^3$ Wasserstoff, $C_{1-6}$-Alkyl, Trifluormethyl oder Halogen und einer der Reste $R^2$ und $R^3$ Trifluormethyl oder $C_{1-6}$-Alkyl darstellt, $R^4$ und $R^5$ Wasserstoff, $C_{1-10}$-Alkyl, $C_{1-10}$-Alkoxy oder Halogen; $R^6$ Wasserstoff, $C_{1-6}$-Alkyl oder einen Rest $-OR^7$; $R^7$ Wasserstoff, $C_{1-6}$-Alkyl, $C_{1-6}$-Alkenoyl, Benzoyl, p-Nitrobenzoyl, Toluoyl oder Phenylacetyl; $R^8$ Wasserstoff oder $C_{1-6}$-Alkyl darstellt, und mehrere anwesende Reste $R^7$ oder $R^8$ untereinander verschieden sein können.

EP-A-176 033 offenbart Isoxazolcarbonsäure-Derivate, die bei der topischen und systemischen Therapie von Praekanzerosen und Karzinomen sowie bei der Behandlung von Akne, Psoriasis und rheumatischen Erkrankungen eingesetzt werden können. Für gleichartige Anwendung offenbaren EP-A-84 667 und EP-A-176 032 Phenylethylen-Derivate bzw. Tetralin-Derivate.

EP-A-98 591 beschreibt heterocyclische Verbindungen zur Anwendung als Rodentizide, die einen substituierten Pyridylrest aufweisen können.

Die Tetrahydronaphthalin- und Indanderivate der vorliegenden Erfindung besitzen demgegenüber einen unsubstituierten Pyridylrest an entsprechender Stelle.

Die Bezeichnung $C_{1-6}$ bezieht sich auf Gruppen mit 1-6 C-Atomen. Alkyl- und Alkoxygruppen können geradkettig oder verzweigt sein, wie Methyl, Aethyl, Propyl, Isopropyl, Butyl, sek.-Butyl bzw. Methoxy, Aethoxy, Propoxy, Isopropoxy, Butoxy und sek.-Butoxy. Alkyl- und Alkoxygruppen $R^4$ und $R^5$ enthalten bis zu 10 C-Atome, wie Octyl, Nonyl, Decyl und 2,2-Dimethyloctyl bzw. Octyloxy, Nonyloxy, Decyloxy und 2,2-Dimethyloctyloxy. Beispiele von $C_{1-6}$-Alkanoyloxygruppern sind Acetoxy, Propionyloxy, Butyryloxy, Pivaloyloxy und Caproyloxy.

Die Verbindungen der Formel I können als trans- oder cis-Isomere oder cis/trans-Isomerengemische vorliegen. Im allgemeinen sind die trans-Verbindungen der Formel I bevorzugt.

Von den Verbindungen der Formel I sind weiterhin diejenigen bevorzugt, in denen X und Y eine Gruppe $>C(CH_3)_2$ und Z $-CH_2-CH_2-$ darstellen. In Bezug auf die Substituenten $R^2$ und $R^3$ sind Wasserstoff für $R^2$ und $C_{1-6}$-Alkyl, insbesondere Methyl für $R^3$ bevorzugt. $R^4$ ist vorzugsweise Wasserstoff oder Alkyl oder Alkoxy mit bis zu 10 C-Atomen. $R^5$ und $R^6$ sind vorzugsweise Wasserstoff.

Die Erfindung betrifft weiterhin ein Verfahren zur Herstellung der Verbindungen der Formel I, pharmazeutische Präparate auf Basis der Verbindungen der Formel I, die Verbindungen der Formel I bei der Behandlung und Prophylaxe von Neoplasien und Dermatosen sowie der Verwendung der Verbindungen der Formel I bei der Herstellung von pharmazeutischen Präparaten zur Behandlung und Prophylaxe solcher Erkrankungen.

Die Verbindungen der Formel I können erfindungsgemäss dadurch erhalten werden, dass man eine Verbindung der allgemeinen Formel

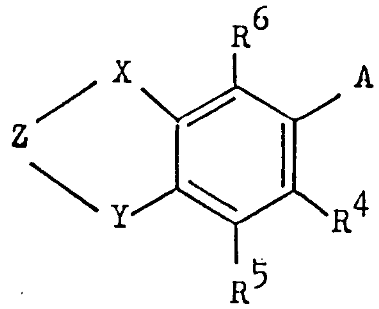

II

mit einer Verbindung der allgemeinen Formel

$R^1$-B

umsetzt,
wobei entweder
A einen der Reste $-CH(R^3)P^+(Q)_3Y^-$ oder $-CH(R^3)-P(O)(OAlk)_2$ darstellt, und B einen Rest $R^{21}$-CO- darstellt; oder
A einen Rest $R^{31}$-CO- und B einen der Reste $-CH(R^2)P^+(Q)_3Y^-$ , oder $-CH(R^2)-P(O)(OAlk)_2$ oder $-CH(R^{21})$-MgHal darstellt; oder
A einen Rest $-CH(R^{31})$MgHal und B einen Rest $R^2$-CO-darstellt; wobei in den obigen Formeln Q Aryl; $Y^-$ das Anion einer organischen oder anorganischen Säure; Alk eine $C_{1-6}$-Alkylgruppe; Hal Halogen; $R^{21}$ und $R^{31}$ Wasserstoff, Trifluormethyl oder $C_{1-6}$-Alkyl ist; und $R^1$, $R^2$, $R^3$, $R^4$, $R^5$, X, Y und Z die oben angegebene Bedeutung haben.

Die Umsetzung der Verbindungen der Formeln II und $R^1$-B kann nach den bekannten Methoden der Wittig-, Horner- oder Grignard-Reaktion durchgeführt werden.

Bei der Wittig-Reaktion, d.h., bei Verwendung einer Verbindung der Formel II mit A = $-CH(R^3)P^+(Q)_3Y^-$ oder der Formel $R^1$-B mit B = $-CH(R^2)P^+(Q)_3Y^-$, werden die Komponenten in Gegenwart eines säurebindenden Mittels, z.B. in Gegenwart einer starken Base, wie z.B. Butyllithium, Natriumhydrid oder dem Natriumsalz von Dimethylsulfoxyd, vornehmlich aber in Gegenwart eines gegebenenfalls durch niederes Alkyl substituierten Aethylenoxyds, wie 1,2-Butylenoxyd. gegebenenfalls in einem Lösungsmittel, z.B. in einem Aether, wie Diäthyläther oder Tetrahydrofuran, oder in einem aromatischen Kohlenwasserstoff, wie Benzol, in einem zwischen Raumtemperatur und dem Siedepunkt des Reaktionsgemisches liegenden Temperaturbereich miteinander umgesetzt.

Von den anorganischen Säureanionen $Y^-$ ist das Chlor- und Brom-ion oder das Hydrosulfat-ion, von den organischen Säureanionen ist das Tosyloxy-ion bevorzugt. Der Arylrest Q ist vorzugsweise ein Phenylrest oder ein substituierter Phenylrest, wie p-Tolyl.

Bei der Horner-Reaktion, d.h. bei Verwendung einer Verbindung der Formel II mit A = $-CH(R^3)-P(O)-(OAlk)_2$ oder der Formel $R^1$-B mit B = $-CH(R^2)-P(O)(OAlk)_2$ werden die Komponenten mit Hilfe einer Base und vorzugsweise in Gegenwart eines inerten organischen Lösungsmittels, z.B. mit Hilfe von Natriumhydrid in Benzol, Toluol, Dimethylformamid. DMSO, Tetrahydrofuran, Dioxan oder 1,2-Dimethoxyalkan, oder auch mit Hilfe eines Natriumalkoholates in einem Alkanol, z.B. Natriummethylat in Methanol, in einem zwischen 0° und dem Siedepunkt des Reaktionsgemisches liegenden Temperaturbereich kondensiert.

Die Alkoxyreste OAlk sind vornehmlich niedere Alkoxyreste mit 1-6 Kohlenstoffatomen, wie Methoxy oder Aethoxy.

Die Umsetzung einer Verbindung der Formel II mit A = $-CH(R^{31})$MgHal bzw. der Formel $R^1$-B mit B = $-CH(R^{21})$MgHal kann in an sich bekannter Weise unter den Bedingungen einer Grignard-Reaktion vorgenommen werden, z.B. in einem Aether, wie Diäthyläther oder Tetrahydrofuran, bei Raumtemperatur und anschliessender Wasserabspaltung mit sauren Agentien, z.B. mit organischen Säuren, wie p-Toluolsulfosäure.

Die Verbindungen der Formel I können wie gesagt in trans- oder cis-Form vorliegen. Bei der Herstellung fallen sie mehrheitlich in der trans-Form an. Gegebenenfalls anfallende cis-Anteile können in an sich bekannter Weise, falls erwünscht, abgetrennt werden.

Die Ausgangsverbindungen der Formeln II und $R^1$-B können, soweit ihre Herstellung nicht bekannt oder nachstehend beschrieben ist, in Analogie zu bekannten oder den nachstehend beschriebenen Methoden hergestellt werden.

Die Verbindungen der Formel I sind therapeutisch wirksam. Sie besitzen insbesondere anti-seborrhoische, anti-keratinisierende, anti-neoplastische und anti-allergische/anti-inflammatorische Wirksamkeit, die mit den nachstehend beschriebenen Versuchsanordnungen gezeigt werden kann:

A) Die anti-keratinisierende Wirkung kann nach dem folgenden Prozedere am Rhinomausmodell bestimmt werden. Die Haut der Rhinomaus zeichnet sich durch das Vorhandensein von mit Keratin angefüllten Utriculi der Epidermis und subkutanen Zysten aus, welche beide von Haarfollikeln herrühren. Die Verabreichung von Retinoiden führt zu einer Hyperproliferation der Epidermis und der epithelialen Auskleidung der Utriculi. Die Verdickung der Epidermis und die Reduktion der Grösse der Utriculi führen zu einer Normalisierung der veränderten Struktur der Epithelschicht. Die tägliche topische Applikation von 0,1 ml/cm$^2$ Haut der Rhinomaus von einer 3%igen Acetonlösung einer wirksamen Versuchsverbindung während 3 Wochen bzw. die dreimalige wöchentliche orale Verabreichung in Arachisöl während 3 Wochen führt zu einer signifikanten Proliferation der Epidermis und markanten Verkleinerung der mit Keratin angefüllten Utriculi.

B) Die Wirkung bei der Verhütung chemisch induzierter Mammatumoren kann nach dem folgenden Prozedere bestimmt werden. Weibliche Sprague-Dawley-Ratten werden unter Temperatur- und Licht-kontrollierten Bedingungen gehalten, bei freiem Zugang zu Trinkwasser und Futter. Im Alter von 50 Tagen werden jeder Ratte 15 mg in Arachisöl gelöstes Dimethylbenz(a)anthracen mittels einer Magensonde verabreicht. Die Behandlung mit den Versuchs-Verbindungen beginnt 1 Tag nach der Verabreichung des Karzinogens. Die Körpergewichte der Versuchstiere werden aufgezeichnet und die Tumoren wöchentlich palpiert und mit einer Schublehre ausgemessen. Die Volumina werden nach der Formel $\frac{D}{2} \cdot d^2$ berechnet, wobei D der grössere und d der kleinere Durchmesser des Tumorellipsoids darstellt. Der Versuch wird nach 11 Wochen beendet und ausgewertet. In diesem Versuch werden neben den 30 Kontrolltieren, welche ausschliesslich normales Futter erhalten, die folgenden zwei Gruppen von Versuchstieren eingesetzt:

1. 33 Ratten, denen mit dem Futter vermischt täglich 30 mg/kg Versuchs-Verbindung verabreicht werden.
2. 36 Ratten, denen mit dem Futter vermischt täglich 90 mg/kg Versuchs-Verbindung verabreicht werden.

C) Die Wirkung auf Tumoren kann weiterhin am transplantablen Chondrosarkom der Ratte nach der folgenden Methode bestimmt werden. Der feste Tumor eines Spendertiers wird fein zerteilt und in Phosphatpuffer/Kochsalzlösung suspendiert. 0,5 ml des 30%igen Tumorbreis wird Albinoratten subkutan implantiert.

Die transplantierten Ratten werden auf Versuchsgruppen von je 8 Tieren verteilt. Die Versuchsverbindungen werden in Arachisöl suspendiert und während 24 Tagen fünfmal wöchentlich mittels Schlundsonde oral verabreicht. Die Tumoren werden am Tag 24 exzidiert und gewogen. Die Resultate werden im Quotienten C/T ausgedrückt, der sich wie folgt berechnet:

$$C/T = \frac{\text{Mittleres Tumorgewicht der Kontrolle}}{\text{Mittleres Tumorgewicht der Behandelten.}}$$

D) Die antimetaplastische Wirkung kann auch nach der folgenden Methode bei Ratten bestimmt werden. Weibliche Holtzmann-Ratten mit einem Gewicht von ca. 100 g werden nach einer Eingewöhnungszeit von 8 Tagen unter Thiogenalnarkose ovarektomiert und nach weiteren 14 Tagen in den Versuch genommen. Je zwei Tiere werden in einem Käfig untergebracht mit freiem Zugang zu Futter, das ca. 2000 IE analytisch bestimmtes Vitamin A enthält. Vor der oralen Verabreichung der Testverbindung werden die Tiere an 6 aufeinanderfolgenden Tagen täglich mit 1 µg Oestradiolbenzoat und 250 µg Testosteronpropionat, gelöst in 0,1 ml Sesamöl, subkutan behandelt. Die parenterale Hormonapplikation führt zur Ausbildung eines reinen Schollenstadiums im Vaginalbereich, d.h. einer squamösen Metaplasie. 2 Tage nach der oralen Verabreichung der Testsubstanz wird das Reaktionsergebnis wiederum am Vaginalepithel abgelesen. Für die Berechnung der mittleren wirksamen Dosen wird die Flächenmethode nach Behrens und Kärber herangezogen.

E) Die Wirkung der Verbindungen I auf die Sebum-Ausscheidung bei Ratten wurde nach dem folgenden Prozedere bestimmt. Männliche Ratten von ungefähr 50-60 g Körpergewicht wurden im Alter von 21-22 Tagen kastriert. Eine Woche nach dieser Operation wurden die Ratten in einer Reinigungslösung gewaschen, um Sebum, das vor der Testperiode ausgeschieden wurde, zu entfernen. Einer Gruppe von Ratten wurden lediglich die verwendeten Trägermaterialien verabreicht. Eine weitere Gruppe von Ratten

erhielt gleichzeitig auch noch 100 µg Testosteronpropionat in 0,2 ml Sesamöl pro Ratte und Tag. Einer weiteren Gruppe von Ratten wurde täglich pro Ratte 100 µg Testosteronpropionat in 0,2 ml Sesamöl subkutan und die Versuchsverbindungen in verschiedenen Dosen in 0,2 ml Propylenglykol oral verabreicht. Die Ratten wurden während 14 Tagen so behandelt. Am 15. Tag wurde das Sebum von der Hautoberfläche und dem Pelz entfernt, indem der ganze Körper der Versuchstiere in ein bestimmtes Volumen Aceton getaucht und während 2 Minuten darin gebadet wurde. Ein Aliquot des Lösungsmittelbades wurde eingedampft, und der feste Rückstand gravimetrisch bestimmt. Die Inhibierung der Testosteron-stimulierten Zunahme der Sebum-Ausscheidung im Vergleich zu den entsprechenden Werten von nur mit Testosteronpropionat behandelten Ratten wurde als Mass für die Wirkung verwendet.

Die Verbindungen der Formel I können zur topischen und systemischen Therapie von benignen und malignen Neoplasien, von prämalignen Läsionen, sowie ferner auch zur systemischen und topischen Prophylaxe der genannten Affektionen verwendet werden.

Sie sind des weiteren für die topische und systemische Therapie von Akne, Psoriasis und anderen mit einer verstärkten oder pathologisch veränderten Verhornung einhergehenden Dermatosen, wie auch von entzündlichen und allergischen dermatologischen Affektionen geeignet. Die Verfahrensprodukte der Formel I können ferner auch zur Bekämpfung von Schleimhauterkrankungen mit entzündlichen oder degenerativen bzw. metaplastischen Veränderungen eingesetzt werden.

Die Mittel können enteral, parenteral oder topisch verabreicht werden. Für die enterale Applikation eignen sich z.B. Mittel in Form von Tabletten, Kapseln, Dragees, Sirupen, Suspensionen, Lösungen und Suppositorien. Für die parenterale Applikation sind Mittel in Form von Infusionsoder Injektionslösungen geeignet.

Die Dosierungen, in denen die Präparate verabreicht werden, können je nach Anwendungsart und Anwendungsweg sowie nach den Bedürfnissen der Patienten variieren. Im allgemeinen kommen für den Erwachsenen tägliche Dosen von etwa 0,1-50 mg/kg, vorzugsweise 1-15 mg/kg in Betracht.

Die Präparate können in einer oder mehreren Dosierungen verabreicht werden. Eine bevorzugte Darreichungsform sind Kapseln mit einem Gehalt von ca. 5-200 mg Wirkstoff.

Die Präparate können inerte oder auch pharmakodynamisch aktive Zusätze enthalten. Tabletten oder Granula z.B. können eine Reihe von Bindemitteln, Füllstoffen, Trägersubstanzen oder Verdünnungsmitteln enthalten. Flüssige Präparate können beispielsweise in Form einer sterilen, mit Wasser mischbaren Lösung vorliegen. Kapseln können neben dem Wirkstoff zusätzlich ein Füllmaterial oder Verdickungsmittel enthalten. Des weiteren können geschmacksverbessernde Zusätze, sowie die üblicherweise als Konservierungs-, Stabilisierungs-, Feuchthalte- und Emulgiermittel verwendeten Stoffe, ferner auch Salze zur Veränderung des osmotischen Druckes, Puffer und andere Zusätze vorhanden sein.

Die vorstehend erwähnten Trägersubstanzen und Verdünnungsmittel können aus organischen oder anorganischen Stoffen, z.B. aus Wasser, Gelatine, Milchzucker, Stärke, Magnesiumstearat, Talkum, Gummi arabicum, Polyalkylenglykolen und dgl. bestehen. Voraussetzung ist, dass alle bei der Herstellung der Präparate verwendeten Hilfsstoffe untoxisch sind.

Zur topischen Anwendung werden die Wirkstoffe zweckmässig in Form von Salben, Tinkturen, Crèmen, Lösungen, Lotionen, Sprays, Suspensionen und dgl. verwendet. Bevorzugt sind Salben und Crèmen sowie Lösungen. Diese zur topischen Anwendung bestimmten Präparate können dadurch hergestellt werden, dass man die Wirkstoffe nichttoxischen, inerten, für topische Behandlung geeigneten, an sich in solchen Präparaten üblichen festen oder flüssigen Trägern zumischt.

Für die topische Anwendung sind zweckmässig ca. 0,1-5%ige, vorzugsweise 0,3-2%ige Lösungen, sowie ca. 0,1-5%ige, vorzugsweise ca. 0,3-2%ige Salben oder Crèmen geeignet.

Den Präparaten kann gegebenenfalls ein Antioxydationsmittel, z.B. Tocopherol, N-Methyl-γ-tocopheramin sowie butyliertes Hydroxyanisol oder butyliertes Hydroxytoluol beigemischt sein.

Das nachstehende Beispiel erläutert die Erfindung weiter. Die Temperaturen sind in Celsiusgraden angegeben.

Beispiel

378 g [1-(5,6,7,8-Tetrahydro-5,5,8,8-tetramethyl -2-naphthyl)äthyl]triphenylphosphoniumbromid werden in 4 l Butylenoxid suspendiert. Nach Zugabe von 51 ml 4-Pyridin-carbaldehyd kocht man das Gemisch 1,5 Stunden am Rückfluss. Nach dem Abkühlen dampft man den grössten Teil des Lösungsmittels im Wasserstrahlvakuum ab, giesst den Rückstand in 1,5 l eines Methanol/Wasser-Gemisches (Verhältnis 6:4) und extrahiert mehrfach mit Hexan. Die organische Phase wird dreimal mit Wasser gewaschen und nach dem Trocknen über Natriumsulfat eingedampft. Nach Flash-Chromatographie (Eluierungsmittel Hexan/Aether = 2:1) und Umkristallisation aus Hexan erhält man 216 g 4-[(E)-2-(5,6,7,8-Tetrahydro-5,5,8,8-tetramethyl -2-naphthyl)propenyl]pyridin in farblosen Kristallen, Schmelzpunkt 84-85°.

Analog erhält man

3-[(E)-2-(5,6,7,8-Tetrahydro-5,5,8,8-tetramethyl-2-naphthyl)propenyl]pyridin, Schmelzpunkt 93-95°,

2-[(E)-2-(5,6,7,8-Tetrahydro-5,5,8,8-tetramethyl-2-naphthyl)propenyl]pyridin, Schmelzpunkt 77-79°,

## Patentansprüche

### Patentansprüche für folgende Vertragsstaaten : BE, CH, DE, FR, GB, IT, LI, LU, NL, SE

1. Verbindungen der allgemeinen Formel

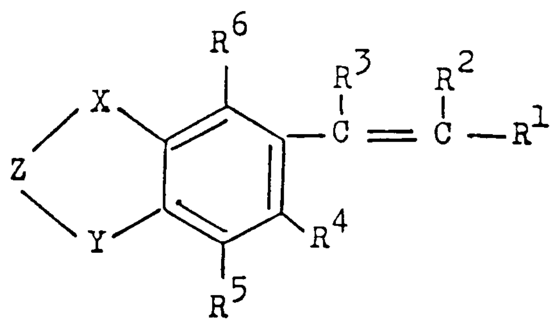

I

worin X und Y $-CH_2-$ oder $>C(CH_3)_2$; Z einen Rest $-CHR^8-$, >CO, $>CR^8OR^7$, $-CHR^8-CHR^8-$, $-CHOR^7-CH_2$, $-CO-CHOR^7-$ oder $-CHOR^7-CHOR^7-$darstellt; $R^1$ Pyridyl darstellt, $R^2$ und $R^3$ Wasserstoff, $C_{1-6}$-Alkyl, Trifluormethyl oder Halogen und einer der Reste $R^2$ und $R^3$ Trifluormethyl oder $C_{1-6}$-Alkyl darstellt, $R^4$ und $R^5$ Wasserstoff, $C_{1-10}$-Alkyl, $C_{1-10}$-Alkoxy oder Halogen; $R^6$ Wasserstoff, $C_{1-6}$-Alkyl oder einen Rest $-OR^7$; $R^7$ Wasserstoff, $C_{1-6}$-Alkyl, $C_{1-6}$-Alkanoyl, Benzoyl, p-Nitrobenzoyl, Toluoyl oder Phenylacetyl; $R^8$ Wasserstoff oder $C_{1-6}$-Alkyl darstellt, und mehrere anwesende Reste $R^7$ oder $R^8$ untereinander verschieden sein können.

2. Verbindungen gemäss Anspruch 1, in denen X und Y $>C(CH_3)_2$; Z $-CH_2-CH_2-$, $R^1$ Pyridyl; $R^2$, $R^5$ und $R^6$ Wasserstoff; und $R^4$ Wasserstoff oder $C_{1-10}$-Alkyl oder $C_{1-10}$-Alkoxy darstellen.

3. 4-[(E)-2-(5, 6, 7, 8-Tetrahydro-5, 5, 8, 8-tetramethyl-2-naphthyl) pro-penyl] pyridin.

4. 3-[(E)-2-(5, 6, 7, 8-Tetrahydro-5, 5, 8, 8-tetramethyl-2-naphthyl) propenyl] pyridin.

5. 2-[(E)-2-(5, 6, 7, 8-Tetrahydro-5, 5, 8, 8-tetramethyl-2-naphthyl) propenyl] pyridin.

6. Die Verbindungen gemäss den Ansprüchen 1-5 zur Verwendung als Heilmittel.

7. Verwendung einer Verbindung gemäss den Ansprüchen 1-5 zur Herstellung von pharmazeutischen Präparaten für die Behandlung von Neoplasien und Dermatosen.

8. Pharmazeutische Präparate entahltend eine Verbindung gemäss den Ansprüchen 1-5 und einen pharmazeutischen Trägerstoff.

9. Pharmazeutische Präparate gemäss Anspruch 8, enthaltend 5-200 mg einer Verbindung der Ansprüche 1-5 pro Dosierungseinheit.

10. Verfahren zur Herstellung der Verbindungen der Ansprüche 1-5 dadurch gekennzeichnet, dass man eine Verbindung der allgemeinen Formel

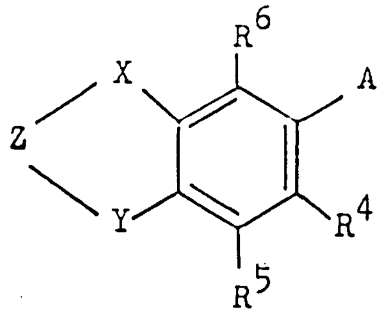

II

mit einer Verbindung der allgemeinen Formel

$R^1$-B

umsetzt,
wobei entweder
A einen der Reste $-CH(R^3)P^+(Q)_3Y^-$ oder $-CH(R^3)-P(O)(OAlk)_2$ darstellt, und B einen Rest $R^{21}$-CO-darstellt; oder
A einen Rest $R^{31}$-CO- und B einen der Reste $-CH(R^2)P^+(Q)_3Y^-$, oder $-CH(R^2)-P(O)(OAlk)_2$ oder -CH-($R^{21}$)MgHal darstellt; oder
A einen Rest -CH($R^{31}$) MgHal und B einen Rest $R^2$-CO- darstellt; wobei in den obigen Formeln Q Aryl; $Y^-$ das Anion einer organischen oder anorganischen Säure; Alk eine $C_{1-6}$-Alkylgruppe; Hal Halogen; $R^{21}$ und $R^{31}$ Wasserstoff, Trifluormethyl oder $C_{1-6}$-Alkyl ist; und $R^1$, $R^2$, $R^3$, $R^4$, $R^5$, X, Y und Z die oben angegebene Bedeutung haben.

**Patentansprüche für folgende Vertragsstaaten : AT, ES, GR**

**1.** Verfahren zur Herstellung von Verbindungen der allgemeinen Formel

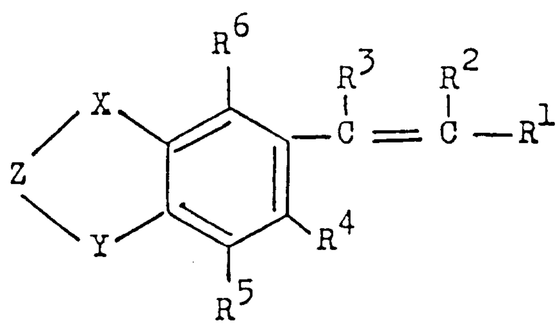

I

worin X und Y $-CH_2-$ oder $>C(CH_3)_2$; Z einen Rest $-CHR^8-$, >CO, $>CR^8OR^7$, $-CHR^8-CHR^8-$, $-CHOR^7-CH_2-$, $-CO-CHOR^7-$ oder $-CHOR^7-CHOR^7-$darstellt; $R^1$ Pyridyl darstellt, $R^2$ und $R^3$ Wasserstoff, $C_{1-6}$-Alkyl, Trifluormethyl oder Halogen und einer der Reste $R^2$ und $R^3$ Trifluormethyl oder $C_{1-6}$-Alkyl darstellt, $R^4$ und $R^5$ Wasserstoff, $C_{1-10}$-Alkyl, $C_{1-10}$-Alkoxy oder Halogen; $R^6$ Wasserstoff, $C_{1-6}$-Alkyl oder einen Rest $-OR^7$; $R^7$ Wasserstoff, $C_{1-6}$-Alkyl, $C_{1-6}$-Alkanoyl, Benzoyl, p-Nitrobenzoyl, Toluoyl oder Phenylacetyl; $R^8$ Wasserstoff oder $C_{1-6}$-Alkyl darstellt, und mehrere anwesende Reste $R^7$ oder $R^8$ untereinander verschieden sein können,
dadurch gekennzeichnet, dass man eine Verbindung der allgemeinen Formel

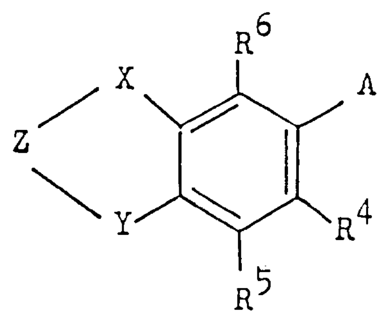

II

mit einer Verbindung der allgemeinen Formel

$R^1$-B

umsetzt,
wobei entweder
A einen der Reste $-CH(R^3)P^+(Q)_3Y^-$ oder $-CH(R^3)-P(O)(OAlk)_2$ darstellt, und B einen Rest $R^{21}$-CO- darstellt; oder
A einen Rest $R^{31}$-CO- und B einen der Reste $-CH(R^2)P^+(Q)_3Y^-$, oder $-CH(R^2)-P(O)(OAlk)_2$ oder $-CH(R^{21})MgHal$ darstellt; oder
A einen Rest $-CH(R^{31})MgHal$ und B einen Rest $R^2$-CO- darstellt; wobei in den obigen Formeln Q Aryl; $Y^-$ das Anion einer organischen oder anorganischen Säure; Alk eine $C_{1-6}$-Alkylgruppe; Hal Halogen; $R^{21}$ und $R^{31}$ Wasserstoff, Trifluormethyl oder $C_{1-6}$-Alkyl ist; und $R^1$, $R^2$, $R^3$, $R^4$, $R^5$, X, Y und Z die oben angegebene Bedeutung haben.

2. Verfahren nach Anspruch 1 zur Herstellung von Verbindungen der Formel I, in deren X und Y $>C(CH_3)_2$; Z $-CH_2-CH_2-$, $R^1$ Pyridyl; $R^2$, $R^5$ und $R^6$ Wasserstoff; und $R^4$ Wasserstoff oder $C_{1-10}$ Alkyl oder $C_{1-10}$ Alkoxy darstellen.

3. Verfahren nach Anspruch 1 zur Herstellung von 4-[(E)-2-(5, 6, 7, 8-Tetrahydro-5, 5, 8, 8-tetramethyl-2-naphthyl)-propenyl] pyridin.

4. Verfahren nach Anspruch 1 zur Herstellung von 3-[(E)-2-(5, 6, 7, 8-Tetrahydro-5, 5, 8, 8-tetramethyl-2-naphthyl)propenyl] pyridin.

5. Verfahren nach Anspruch 2 zur Herstellung von 2-[(E)-2-(5,6,7,8-Tetrahydro-5, 5, 8, 8-tetramethyl-2-naphthyl)-propenyl]pyridin.

6. Verwendung einer Verbindung der Formel I zur Herstellung von pharmazeutischen Präparaten für die Behandlung von Neoplasien und Dermatosen.

7. Verfahren zur Herstellung pharmazeutischer Präparate, enthaltend eine Verbindung der Formel I und einen pharmazeutischen Trägerstoff.

## Claims

**Claims for the following Contracting States : BE, CH, DE, FR, GB, IT, LI, LU, NL, SE**

**1.** Compounds of the general formula

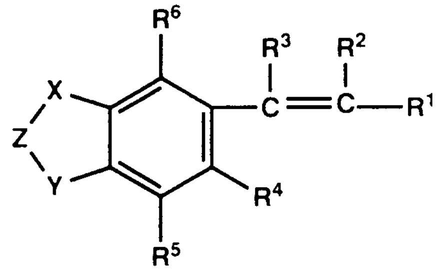

I

wherein X and Y represent $-CH_2-$ or $>C(CH_3)_2$; Z represents a residue $-CHR^8-$, $>CO$, $>CR^8OR^7$, $-CHR^8-CHR^8-$, $-CHOR^7-CH_2-$, $-CO-CHOR^7$ or $-CHOR^7-CHOR^7-$; $R^1$ represents pyridyl, $R^2$ and $R^3$ represent hydrogen, $C_{1-6}$-alkyl, trifluoromethyl or halogen and one of the residues $R^2$ and $R^3$ represents trifluoromethyl or $C_{1-6}$-alkyl, $R^4$ and $R^5$ represent hydrogen, $C_{1-10}$-alkyl, $C_{1-10}$-alkoxy or halogen; $R^6$ represents hydrogen, $C_{1-6}$-alkyl or a residue $-OR^7$; $R^7$ represents hydrogen, $C_{1-6}$-alkyl, $C_{1-6}$-alkanoyl, benzoyl, p-nitrobenzoyl, toluoyl or phenylacetyl; $R^8$ represents hydrogen or $C_{1-6}$-alkyl; and several residues $R^7$ or $R^8$ present can be different from one another.

**2.** Compounds in accordance with claim 1 in which X and Y represent $>C(CH_3)_2$; Z represent $-CH_2-CH_2-$, $R^1$ represents pyridyl; $R^2$, $R^5$ and $R^6$ represent hydrogen; and $R^4$ represents hydrogen or $C_{1-10}$-alkyl or $C_{1-10}$-alkoxy.

**3.** 4-[(E)2-(5,6,7,8-Tetrahydro-5,5,8,8-tetramethyl-2-naphthyl)propenyl]pyridine.

**4.** 3-[(E)-2-(5,6,7,8-Tetrahydro-5,5,8,8-tetramethyl-2-naphthyl)propenyl]pyridine.

**5.** 2-[(E)-2-(5,6,7,8-Tetrahydro-5,5,8,8-tetramethyl-2-naphthyl)-propenyl]pyridine.

**6.** The compounds in accordance with claims 1-5 for use as medicaments.

**7.** The use of the compounds in accordance with claims 1-5 for the manufacture of pharmaceutical preparations for the treatment of neoplasms and dermatoses.

**8.** Pharmaceutical preparations containing a compound in accordance with claims 1-5 and a pharmaceutical carrier material.

**9.** Pharmaceutical preparations in accordance with claim 8 containing 5-200 mg of a compound of claims 1-5 per dosage unit.

**10.** A process for the manufacture of the compounds of claims 1-5, characterized by reacting a compound of the general formula

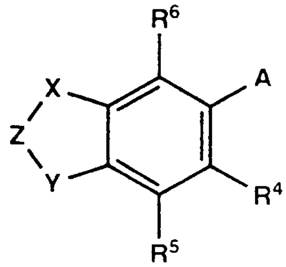

II

with a compound of the general formula

$R^1$-B

in which either
A represents the residue $-CH(R^3)P^+(Q)_3Y^-$ or $-CH(R3)-P(O)(OAlk)_2$ and B represents a residue $R^{21}$-CO-; or
A represents a residue $R^{31}$-CO- and B represents one of the residues $-CH(R^2)P^+(Q)_3Y^-$, or $-CH(R^2)-P-(O)(OAlk)_2$ or $-CH(R^{21})MgHal$; or
A represents a residue $-CH(R^{31})MgHal$ and B represents a residue $R^2$-CO-; whereby in the above formulae Q is aryl; Y- is the anion of an organic or inorganic acid; Alk is a $C_{1-6}$-alkyl group; Hal is halogen; $R^{21}$ and $R^{31}$ are hydrogen, trifluoromethyl or $C_{1-6}$-alkyl; and $R^1$, $R^2$, $R^3$, $R^4$, $R^5$, X, Y and Z have the significance given above.

**Claims for the following Contracting States : AT, ES, GR**

**1.** A process for the manufacture of compounds of the general formula.

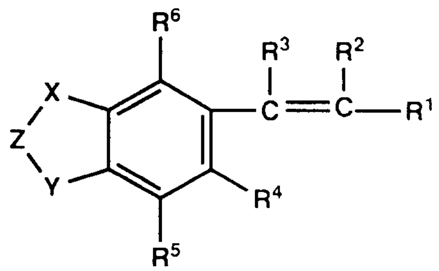

I

wherein X and Y represent $-CH_2-$ or $>C(CH_3)_2$; Z represents a residue $-CHR^8-$, >CO, $>CR^8OR^7$, $-CHR^8-CHR^8-$, $-CHOR^7-CH_2-$, $-CO-CHOR^7-$ or $-CHOR^7-CHOR^7-$; $R^1$ represents pyridyl, $R^2$ and $R^3$ represent hydrogen, $C_{1-6}$-alkyl, trifluoromethyl or halogen and one of the residues $R^2$ and $R^3$ represents trifluoromethyl or $C_{1-6}$-alkyl, $R^4$ and $R^5$ represent hydrogen, $C_{1-10}$-alkyl, $C_{1-10}$-alkoxy or halogen; $R^6$ represents hydrogen, $C_{1-6}$-alkyl or a residue $-OR^7$; $R^7$ represents hydrogen, $C_{1-6}$-alkyl, $C_{1-6}$-alkanoyl, benzoyl, p-nitrobenzoyl, toluoyl or phenylacetyl; $R^8$ represents hydrogen or $C_{1-6}$-alkyl; and several residues $R^7$ or $R^8$ present can be different from one another,
characterized by reacting a compound of the general formula

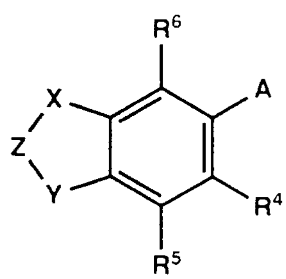

II

with a compound of the general formula

$R^1$-B

in which either
A represents the residue $-CH(R^3)P^+(Q)_3Y^-$ or $-CH(R^3)-P(O)(OAlk)_2$ and B represents a residue $R^{21}$-CO-; or
A represents a residue $R^{31}$-CO- and B represents one of the residues $-CH(R^2)P^+(Q)_3Y^-$, or $-CH(R^2)-P-(O)(OAlk)_2$ or $-CH(R^{21})MgHal$; or
A represents a residue $-CH(R^{31})MgHal$ and B represents a residue $R^2$-CO-; whereby in the above formulae Q is aryl; Y- is the anion of an organic or inorganic acid; Alk is a $C_{1-6}$-alkyl group; Hal is halogen; $R^{21}$ and $R^{31}$ are hydrogen, trifluoromethyl or $C_{1-6}$-alkyl; and $R^1$, $R^2$, $R^3$, $R^4$, $R^5$, X, Y and Z

have the significance given above.

2. A process according to claim 1 for the manufacture of compounds of formula I in which X and Y represent $>C(CH_3)_2$; Z represent $-CH_2-CH_2-$, $R^1$ represents pyridyl; $R^2$, $R^5$ and $R^6$ represent hydrogen; and $R^4$ represents hydrogen or $C_{1-10}$-alkyl or $C_{1-10}$-alkoxy.

3. A process according to claim 1 for the manufacture of 4-[(E)-2-(5,6,7,8-tetrahydro-5,5,8,8-tetramethyl-2-naphthyl)-propenyl]pyridine.

4. A process according to claim 1 for the manufacture of 3-[(E)-2-(5,6,7,8-tetrahydro-5,5,8,8-tetramethyl-2-naphthyl)-propenyl]pyridine.

5. A process according to claim 2 for the manufacture of 2-[(E)-2-(5,6,7,8-tetrahydro-5,5,8,8-tetramethyl-2-naphthyl)-propenyl]pyridine.

6. The use of a compound of formula I for the manufacture of pharmaceutical preparations for the treatment of neoplasms and dermatoses.

7. A process for the manufacture of pharmaceutical preparations containing a compound of formula I and a pharmaceutical carrier material.

**Revendications**
**Revendications pour les Etats contractants suivants : BE, CH, DE, FR, GB, IT, LI, LU, NL, SE**

1. Composés de formule générale

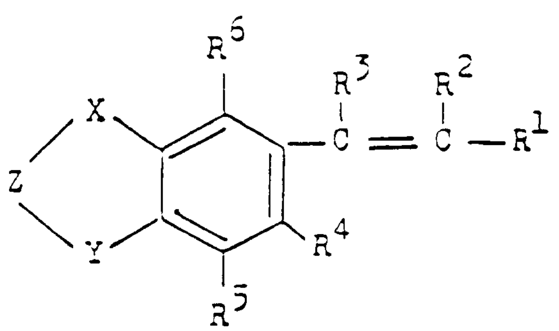

I

ou X et Y représentent $-CH_2-$ ou $>C(CH_3)_2$ ; Z un reste $-CHR^8-$, $>CO$, $>CR^8OR^7$, $-CHR^8-CHR^8-$, $-CHOR^7-CH_2-$, $-CO-CHOR^7-$ ou $-CHOR^7-CHOR^7-$ ; $R^1$ représente le pyridyle, $R^2$ et $R^3$ représentent l'hydrogène, un alkyle en $C_{1-6}$, le trifluorométhyle ou un halogène et l'un des restes $R^2$ et $R^3$ représente le trifluorométhyle ou un alkyle en $C_{1-6}$, $R^4$ et $R^5$ représentent l'hydrogène, un alkyle en $C_{1-10}$, un alcoxy en $C_{1-10}$ ou un halogène ; $R^6$ représente l'hydrogène, un alkyle en $C_{1-6}$ ou un reste $-OR^7$ ; $R^7$ représente l'hydrogène, un alkyle en $C_{1-6}$, un alcanoyle en $C_{1-6}$, le benzoyle, le p-nitrobenzoyle, le toluoyle ou le phénylacétyle ; $R^8$ représente l'hydrogène ou un alkyle en $C_{1-6}$ et plusieurs restes $R^7$ ou $R^8$ présents peuvent être différents les uns des autres.

2. Composés selon la revendication 1, dans lesquels X et Y représentent $>C(CH_3)_2$ ; Z représente $-CH_2-CH_2-$ ; $R^1$ représente le pyridyle ; $R^2$, $R^5$ et $R^6$ représentent l'hydrogène ; et $R^4$ représente l'hydrogène ou un alkyle en $C_{1-10}$ ou un alcoxy en $C_{1-10}$.

3. La 4-[(E)-2-(5,6,7,8-tétrahydro-5,5,8,8-tétraméthyl-2-naphtyl)propényl]pyridine.

4. La 3-[(E)-2-(5,6,7,8-tétrahydro-5,5,8,8-tétraméthyl-2-naphtyl)propényl]pyridine.

5. La 2-[(E)-2-(5,6,7,8-tétrahydro-5,5,8,8-tétraméthyl-2-naphtyl)propényl]pyridine.

6. Les composés selon les revendications 1 à 5 destinés à être utilisés comme médicaments.

**7.** Utilisation d'un composé selon les revendications 1 à 5 pour l'obtention de préparations pharmaceutiques destinées au traitement des néoplasies et des dermatoses.

**8.** Préparations pharmaceutiques contenant un composé selon les revendications 1 à 5 et un support pharmaceutique.

**9.** Préparations pharmaceutiques selon la revendication 8, contenant 5 à 200 mg d'un composé des revendications 1 à 5 par dose unitaire.

**10.** Procédé pour la préparation des composés des revendications 1 à 5, caractérisé en ce que l'on fait réagir un composé de formule générale

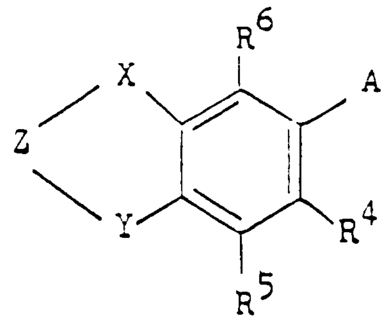

II

avec un composé de formule générale

$R^1$-B

dans laquelle ou bien A représente l'un des restes $-CH(R^3)P^+(Q)_3Y^-$ ou $-CH(R^3)-P(O)(OAlk)_2$ et B un reste $R^{21}$-CO- ;
ou bien A représente un reste $R^{31}$-CO- et B l'un des restes $-CH(R^2)P^+(Q)_3Y^-$ ou $-CH(R^2)-P(O)(OAlk)_2$ ou $-CH-(R^{21})MgHal$ ; ou bien A représente un reste $-CH(R^{31})MgHal$ et B un reste $R^2$-CO- ; dans les formules ci-dessus Q étant un aryle ; $Y^-$ l'anion d'un acide organique ou minéral ; Alk un groupe alkyle en $C_{1-6}$ ; Hal un halogène ; $R^{21}$ et $R^{31}$ l'hydrogène, le trifluorométhyle ou un alkyle en $C_{1-6}$ ; et $R^1$, $R^2$, $R^3$, $R^4$, $R^5$, X et Z ayant les significations indiquées ci-dessus.

**Revendications pour les Etats contractants suivants : AT, ES, GR**

**1.** Procédé pour la préparation des composés de formule générale

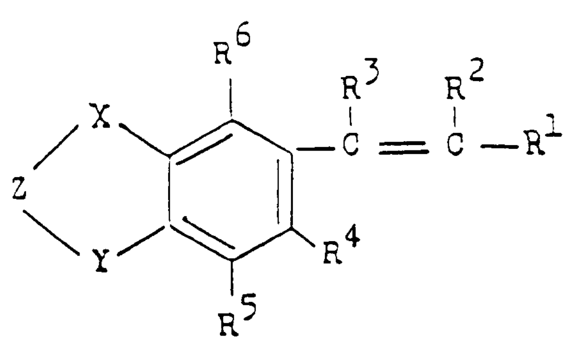

I

où X et Y représentent $-CH_2-$ ou $>C(CH_3)_2$ ; Z un reste $-CHR^8-$, >CO, $>CR^8OR^7$, $-CHR^8-CHR^8-$, $-CHOR^7-CH_2-$, $-CO-CHOR^7-$ ou $-CHOR^7-CHOR^7-$ ; $R^1$ représente le pyridyle, $R^2$ et $R^3$ représentent l'hydrogène, un alkyle en $C_{1-6}$, le trifluorométhyle ou un halogène et l'un des restes $R^2$ et $R^3$ représente le trifluorométhyle ou un alkyle en $C_{1--6}$, $R^4$ et $R^5$ représentent l'hydrogène, un alkyle en $C_{1-10}$, un alcoxy en $C_{1-10}$ ou un halogène ; $R^6$ représente l'hydrogène, un alkyle en $C_{1-6}$ ou un reste $-OR^7$ ; $R^7$ représente l'hydrogène, un alkyle en $C_{1-6}$, un alcanoyle en $C_{1-6}$, le benzoyle, le p-nitrobenzoyle, le toluoyle ou le phénylacétyle ; $R^8$ représente l'hydrogène ou un alkyle en $C_{1-6}$ et plusieurs restes $R^7$ ou $R^8$ présents peuvent être différents les uns des autres, caractérisé en ce que l'on fait réagir un composé de formule générale

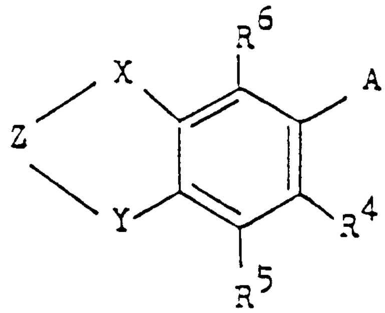

II

avec un composé de formule générale

$R^1$-B

dans laquelle ou bien A représente l'un des restes $-CH(R^3)P^+(Q)_3Y^-$ ou $-CH(R^3)-P(O)(OAlk)_2$ et B un reste $R^{21}$-CO- ; ou bien A représente un reste $R^{31}$-CO- et B l'un des restes $-CH(R^2)P^+(Q)_3Y^-$ ou $-CH-(R^2)-P(O)(OAlk)_2$ ou $-CH-(R^{21})MgHal$ ; ou bien A représente un reste $-CH(R^{31})MgHal$ et B un reste $R^2$-CO- ; dans les formules ci-dessus Q étant un aryle ; $Y^-$ l'anion d'un acide organique ou minéral ; Alk un groupe alkyle en $C_{1-6}$ ; Hal un halogène $R^{21}$ et $R^{31}$ l'hydrogéne, le trifluorométhyle ou un alkyle en $C_{1-6}$ ; et $R^1$, $R^2$, $R^3$, $R^4$, $R^5$, X, Y et Z ayant les significations indiquées ci-dessus.

**2.** Procédé selon la revendication 1, pour la préparation des composés de formule I dans laquelle X et Y représentent $>C(CH_3)_2$ ; Z représente $-CH_2-CH_2-$ ; $R^1$ représente le pyridyle ; $R^2$, $R^5$ et $R^6$ représentent l'hydrogène ; et $R^4$ représente l'hydrogène ou un alkyle en $C_{1-10}$ ou un alcoxy en $C_{1-10}$.

**3.** Procédé selon la revendication 1, pour la préparation de la 4-[(E)-2-(5,6,7,8-tétrahydro-5,5,8, 8-tétraméthyl-2-naphtyl)propényl]pyridine.

**4.** Procédé selon la revendication 1, pour la préparation de la 3-[(E)-2-(5,6,7,8-tétrahydro-5,5,8,8-tétraméthyl-2-naphtyl)propényl]pyridine.

**5.** Procédé selon la revendication 2, pour la préparation de la 2-[(E)-2-(5,6,7,8-tétrahydro-5,5,8, 8-tétraméthyl-2-naphtyl)propényl]pyridine.

**6.** Utilisation d'un composé de formule I pour l'obtention de préparations pharmaceutiques destinées au traitement des néoplasties et des dermatoses.

**7.** Procédé pour l'obtention de préparations pharmaceutiques contenant un composé de formule I et un support pharmaceutique.